(19) **Europäisches Patentamt**

**European Patent Office**

**Office européen des brevets**

(11) **EP 1 043 968 B1**

(12) **FASCICULE DE BREVET EUROPEEN**

(45) Date de publication et mention
de la délivrance du brevet:
**15.09.2004 Bulletin 2004/38**

(51) Int Cl.⁷: **A61K 7/06**, A61K 7/32,
A61K 7/46, A61K 7/48

(21) Numéro de dépôt: **99946386.2**

(22) Date de dépôt: **06.10.1999**

(86) Numéro de dépôt international:
**PCT/IB1999/001635**

(87) Numéro de publication internationale:
**WO 2000/024367 (04.05.2000 Gazette 2000/18)**

(54) **COMPOSITIONS PARFUMANTES ANTIMICROBIENNES**

ANTIMIKROBIELLE DUFTENDE ZUSAMMENSETZUNGEN

ANTIMICROBIAL PERFUME COMPOSITIONS

(84) Etats contractants désignés:
**CH DE ES FR GB IT LI NL**

(30) Priorité: **26.10.1998  CH 215498**

(43) Date de publication de la demande:
**18.10.2000  Bulletin 2000/42**

(73) Titulaire: **FIRMENICH SA**
**1211 Genève 8 (CH)**

(72) Inventeur: **HOLZNER, Günter**
**CH-1212 Grand-Lancy (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
WO-A-92/18100          WO-A-93/25185
WO-A-95/15146          GB-A- 1 401 550
US-A- 5 759 974

## Description

La présente invention a trait au domaine de la parfumerie et de la cosmétique. Elle concerne plus particulièrement le parfumage d'articles destinés aux soins corporels ou capillaires, ainsi que le parfumage de produits fonctionnels.

Elle permet l'élaboration de produits parfumés dont le parfum procure non seulement une agréable odeur, mais s'avère également renforcer, de façon synergique, l'activité antimicrobienne du produit auquel il est incorporé.

De nombreux ingrédients parfumants conventionnels, tels que des huiles essentielles ou des substances odorantes synthétiques, ont été testés pour leurs propriétés antimicrobiennes contre divers microorganismes. Le brevet EP-A1-0 451 889, par exemple, contient un descriptif des connaissances de l'art dans le domaine de l'activité bactéricide des ingrédients parfumants d'origine naturelle ou synthétique. Le brevet EP-A1-0433132, quant à lui, décrit une composition cosmétique contenant des huiles essentielles ayant une activité autre que parfumante, à savoir une activité antibactérienne et/ou antifongique. D'autre part, la demande de brevet WO 93/25185 décrit une composition parfumée contenant un phospholipide cationique, une base parfumante à action antimicrobienne et un alcool gras, composition ayant une activité antimicrobienne mesurée par la méthode du "spray direct". Finalement, il convient de citer la demande WO 98/02044 qui divulgue des compositions ayant un effet désinfectant et étant une combinaison d'un agent tensioactif, un agent chélatant et d'une huile essentielle à action antimicrobienne. Compte tenu de leurs propriétés antimicrobiennes, il n'est pas étonnant de constater que la sélection d'ingrédients parfumants ayant une activité antimicrobienne ainsi que la recherche de composition parfumante possédant à la fois un caractère olfactif et une activité antimicrobienne efficace soient l'objet de nombreuses études.

Toutefois, l'activité microbienne des huiles essentielles ou des substances odorantes, d'origine naturelle ainsi que d'origine synthétique, est relativement faible et pas suffisamment efficace pour lutter contre les microorganismes qui sont ou peuvent entrer en contact avec des personnes. Dans ce contexte, il convient de rappeler que des microorganismes comme des bactéries, levures et champignons se trouvent non seulement sur des surfaces de pratiquement tout objet, mais aussi sur la peau humaine où les microorganismes sont à l'origine de mauvaises odeurs suite à la décomposition de la sueur et autres substances organiques.

Pour de telles raisons, des produits de nettoyage variés ainsi que des produits de soin corporel contiennent souvent, en sus des substances odorantes d'origine diverse, des bactéricides, ce qui augmente d'une façon considérable l'activité microbienne d'un produit donné.

Or, vu les nouvelles tendances des consommateurs vers des produits dits "naturels", il existe un fort besoin en produits de nettoyage et, surtout, de soin corporel qui soient exempts de produits bactéricides ou qui en contiennent moins que les produits qui se trouvent actuellement sur le marché.

La présente demande apporte une nouvelle solution à ce besoin au travers de compositions parfumantes antimicrobiennes associant certains ingrédients parfumants présentant une activité antimicrobienne particulière, avec un ingrédient actif choisi parmi un extrait de pépin de pamplemousse, un extrait de fumeterre, l'acide fumarique ou un ester de l'acide fumarique ou lactique. Certains de ces ingrédients actifs ont été décrits dans des compositions comprenant aussi un parfum.

En particulier, GB 1401550 décrit des compositions déodorantes à base d'ingrédients actifs tels que des esters de l'acide fumarique, et de composés dits « ré-odorants » ou parfums. L'activité déodorante décrite dans ce document est une action qui consiste à faire réagir chimiquement un ingrédient actif avec un composé responsable d'une mauvaise odeur, transformant celui-ci en composé inodorant. Dans ce cas, l'activité déodorante est distincte d'une activité antimicrobienne, cette dernière consistant à inhiber le développement de microorganismes. Ce document décrit une variété d'ingrédients parfumants se concentrant uniquement sur leur propriété non déodorantes, et ne mentionne aucun intérêt particulier pour des ingrédients ayant des propriétés antimicrobiennes.

Par ailleurs, US 5,759,974 décrit l'utilisation d'acide fumarique dans un produit d'entretien comprenant également un parfum. Cependant, aucune restriction ou choix particulier n'est fait quant au parfum utilisé, en particulier aucune mention n'est faite d'un intérêt pour un parfum à action antimicrobienne.

Or la présente invention apporte aujourd'hui une solution particulièrement efficace dans le domaine de l'action antimicrobienne au travers de la combinaison spécifique d'un ingrédient actif avec des ingrédients parfumants particuliers, ayant une activité antimicrobienne mesurée, cette combinaison mettant en évidence une action synergique des composés utilisés.

## Description de l'invention

La présente invention apporte précisément une solution au problème exposé ci-dessus. En conséquence, l'objet de l'invention est une composition parfumante antimicrobienne, caractérisée en ce que ladite composition comprend

- un ingrédient parfumant présentant une activité antimicrobienne d'au moins 80% telle que mesurée par la méthode du "test sur surface agar enduite" (ASCT), par la méthode du "test par diffusion de vapeurs" ou par la "méthode du spray direct" (MSD) ; et
- un ingrédient actif sélectionné parmi un extrait de pépin de pamplemousse, un extrait de fumeterre, l'acide fumarique ou un ester de l'acide fumarique ou lactique.

Selon plusieurs modes d'exécution, la composition parfumante de l'invention peut contenir un ou plusieurs composants choisis parmi des agents tensioactifs, des émollients (adoucissants pour les cheveux et la peau), des agents antimicrobiens ou encore le 2-cyclododécylidène-1-éthanol, composé appartenant à la classe des muscs macrocycliques (origine : Firmenich SA, Genève, Suisse).

L'ingrédient actif de la composition de l'invention est typiquement un extrait de plante ou un composé contenu dans les principes actifs des plantes. Par exemple, l'acide fumarique est présent dans la fumeterre, faisant partie de son principe actif, ainsi que dans beaucoup d'autres plantes. Parmi les esters de l'acide fumarique, il convient de mentionner surtout le fumarate de diéthyle et le fumarate de digéranyle.

Les ingrédients parfumants qui se prêtent à une utilisation dans l'invention présentent une activité antimicrobienne d'au moins 80% telle que mesurée par la méthode "test sur surface agar enduite" (ASCT - Agar Surface Coating Test), par la méthode "test par diffusion de vapeurs" (VPT - Vapeur Phase Test), "la méthode du spray direct" (MSD) ou par une combinaison de ces méthodes.

L'invention concerne également une composition parfumante contenant au moins 40% en poids d'ingrédients parfumants ayant chacun une activité antimicrobienne d'au moins 80% telle que mesurée par l'une des méthodes mentionnées et au moins 0,1% dudit ingrédient actif.

La présente invention dévoile des méthodes de test originales et efficaces permettant de sélectionner des ingrédients parfumants qui ont une action antimicrobienne d'au moins 80%, en plus de leur activité parfumante, et en outre de tester l'efficacité de cette action dans une composition parfumante ou un produit parfumé selon la présente invention. Nous avons en effet découvert qu'une composition parfumante selon l'invention contenant un ingrédient parfumant tel que défini ci-dessus et un extrait de plante ou une molécule correspondant au principe actif de ladite plante, permettaient d'inhiber l'action des microorganismes précités.

De par ses propriétés antimicrobienne et odorante, une composition parfumante élaborée selon l'invention se prête aussi bien aux applications en parfumerie fine qu'au parfumage de produits fonctionnels. C'est ainsi qu'elle peut être employée de façon avantageuse dans le parfumage d'articles divers tels que les savons, les gels de bain ou de douche, les déodorants et antiperspirants corporels, les shampoings et autres produits d'hygiène capillaire. Elle se prête également au parfumage de détergents ou adoucissants textiles, de désodorisants ambiants, de produits d'entretien, de compositions détergentes ou destinées au nettoyage de la vaisselle ou de surfaces variées, ou encore de blocs pour les toilettes ou WC ou de nettoyants pour WC. Il a été constaté de façon surprenante que la présence d'une composition parfumante selon l'invention renforçait l'action bactéricide et/ou fongicide des articles divers, détergents ou adoucissants textiles, produits d'entretien, déodorisants ambiants auxquels ils étaient incorporés.

Les compositions parfumantes de l'invention peuvent contenir d'autres composants ayant un effet positif ou synergique sur l'activité antimicrobienne de ses ingrédients cités plus haut.

Une classe de ces composants optionnellement présents sont les agents tensioactifs de nature variée. Ces agents sont connus de l'homme de métier et incluent des agents tensioactifs anioniques, cationiques, nonioniques, ou amphotériques, ainsi que des phospholipides. Des exemples d'agents tensioactifs qui sont préférés selon l'invention incluent le lauryl pyrrolidone (en vente sous le nom de Surfadone® LP 300 ; origine : ISP, USA), le lauramide DEA (en vente sous le nom de Monoamide® 716 ; origine : Mona Ind., USA), le monolaurate de glycéryle, l'acide lauraminopropionique (en vente sous le nom de Deriphat® 151C ; origine : Henkel, Allemagne), le O-cymen-5-ol (en vente sous le nom de Biosol ; origine ; Osaka Kasei, Japon), le PCA éthyl cocoyl arginate (en vente sous le nom de CAE ; origine ; Ajinomoto, Japon), et l'octoxylglycerol (en vente sous le nom de Sensiva SC 50 ; origine ; Schülke & Mayr, Allemagne). D'autres agents optionnels préférés de l'invention sont les agents tensioactifs zwitterioniques comportant un groupe ammonium quaternaire, comme le ricinolamidopropyle diméthylamine lactate (en vente sous le nom de Mackalene® 216 ; origine: McIntyre, USA) ou le wheat germamidopropyl diméthylamine lactate (Mackalene® 716; origine: McIntyre, USA). Finalement, il est aussi avantageux d'utiliser des phospholipides, par exemple l'hydroxyéthylcétyl dimonium phosphate (en vente sous le nom de Luviquat® mono CP ; origine : BASF, Allemagne), le cocophosphatidylpropylèneglycol dimonium chlorure (phospholipide CDM) ou le cocoamidopropylpropylèneglycol dimonium chlorure phosphate (phospholipide PTC) (origine : Mona Ind. USA).

Une autre classe d'ingrédients optionnellement présents dans les compositions parfumantes de l'invention sont les émollients, substances ayant un effet adoucissant sur la peau et les cheveux. Ces agents sont d'usage courant dans l'art des produits de soin corporel et capillaire et connus de l'homme de métier. En tant qu'émollients préférés, on cite ici le quaternium 80 (en vente sous le nom de Abil® Quat 3474 ; origine Goldschmidt AG., Allemagne), le ricinoléate de glycéryle (en vente sous le nom de Softigen® 701 ; origine : Hüls Chemie, Allemagne) et le lauryl PCA (en vente sous le nom de Laurydone®; origine : VCIB, France). Une classe d'émollients préférée de l'invention sont les esters de l'acide fumarique et de l'acide lactique. Parmi cette classe préférée d'émollients, on cite en tant que substances les plus appréciées de l'invention le lauroyl/myristyl lactylate (en vente sous le nom de Priazul® 2131 ; origine : Unichema, Hollande), le $C_{12}$-$C_{13}$ alkyllactate (en vente sous le nom de Cosmacol ELI®; origine : Condea, Italie), et le lactate de lauryle (en vente sous le nom de Ceraphyl® 31 ; origine : ISP, USA).

Nous avons trouvé d'une façon inattendue que les compositions parfumantes de l'invention montrent en effet une activité antimicrobienne qui est nettement plus prononcée que prévu sur la base du fait que tous les composants pouvant être présents dans cette composition ne possèdent eux-mêmes qu'une activité antimicrobienne faible. En mélangeant les substances mentionnées auparavant d'une façon appropriée, on observe alors un effet synergique, de sorte que l'on obtient des compositions à effet antimicrobien bien équilibré, actif contre toutes les familles de microorganismes courantes dans ce type de produits et consommation, dans la peau ou dans les cheveux. Un tel effet est difficilement atteint avec les compositions parfumantes connues, même si celles-ci contiennent des bactéricides synthétiques ayant un effet antimicrobien très fort.

Dans ce contexte, il convient de mentionner que des bactéricides standard ne sont en général actifs que contre certaines bactéries. Par exemple, les deux bactéricides les plus utilisées dans les produits cosmétiques, à savoir le Triclocarban (3,4,4'-trichlorocarbanilide) et le Triclosan [5-chloro-2-(2,4-dichlorophénoxy)-phénol] sont bien actifs contre les bactéries Gram-positives, mais montrent une activité faible contre les bactéries Gram-négatives, et surtout les *Pseudomonas aeruginosa,* une espèce de bactéries Gram-négatives quasiment omniprésente. De par la diminution de la flore bactérienne Gram-positive, les bactéricides susmentionnés peuvent détruire l'équilibre entre ces bactéries, provoquant ainsi une augmentation du nombre des bactéries Gram-négatives et la formation des mauvaises odeurs.

Les compositions parfumantes de la présente invention, par contre, sont efficaces contre toutes les bactéries ainsi que contre les levures et champignons et présentent un effet antimicrobien bien équilibré.

Par ailleurs, la présente invention permet de préparer des compositions parfumantes qui, en sus des composants définis précédemment, peuvent contenir des agents antimicrobiens d'usage courant et renforcer de façon synergique leur caractère antimicrobien.

Nous avons ainsi constaté que les compositions parfumantes selon l'invention avaient un effet synergique sur l'activité antimicrobienne de substances antimicrobiennes telles que le Zinc-Pyrion® (origine : Pyrion-Chemie, Allemagne) ou la piroctone olamine (origine : Hoechst, Allemagne).

D'autres agents antimicrobiens présentant un effet synergique avec les compositions parfumantes de l'invention sont les bactéricides mentionnés auparavant, c'est-à-dire le Triclocarban et le Triclosan.

Nous avons en particulier observé que l'addition d'une composition parfumante selon la présente invention (en combinaison avec l'acide fumarique) à des produits de soin corporel ou des produits nettoyants permet de considérablement baisser le taux de Triclocarban ou de Triclosan dans lesdits produits à des valeurs d'environ la moitié jusqu'à un quart de la quantité normalement utilisée ; souvent on a aussi observé une activité antimicrobienne accrue contre certaines bactéries, en particulier du type *Pseudomonas,* qu'avec le Triclocarban ou le Triclosan seul.

Nous savons de l'art antérieur cité précédemment que les ingrédients parfumants utilisés dans la préparation d'un parfum peuvent être choisis non seulement pour leur contribution olfactive mais aussi pour leur action antimicrobiologique. Il nous enseigne que cette action est surtout connue comme étant faible. Or l'activité antimicrobienne des articles ou produits définis précédemment, auxquels une composition parfumante élaborée selon la ou les méthodes de l'invention est ajoutée, s'avère être beaucoup plus efficace que celle mentionnée dans l'art antérieur.

Ainsi, que de tels articles ou produits puissent présenter une action contre la flore microbienne aussi efficace, du seul fait qu'on leur incorpore une composition parfumante constituée d'au moins 40% d'ingrédients parfumants actifs selon les méthodes de test de l'invention et un extrait de plantes ou une molécule correspondant au principe actif de cette plante, se révèle tout à fait surprenant. De plus, il a été constaté que les ingrédients parfumants sélectionnés suivant les méthodes de la présente invention, possèdent une action microbicide ou microbiostatique tout à fait remarquable et inattendue. En effet, ils continuent à présenter une activité antimicrobienne efficace malgré des lavages successifs de la surface agar infectée dans la méthode ASCT ou par simple diffusion de leurs vapeurs, sans application directe sur la surface agar infectée dans la méthode VPT.

La composition parfumante selon la présente invention sera ajoutée aux articles ou produits cités plus haut, que l'on désire parfumer, dans des concentrations usuelles dans l'art. Les valeurs de ces concentrations dépendent de la nature de l'article ou produit parfumant fini, ainsi que de l'effet olfactif recherché et l'homme du métier est à même de les choisir en fonction de ces paramètres. Ainsi les articles, produits parfumants ou désodorisants peuvent contenir typiquement de 0,1% à 20% en poids d'une composition parfumante préparée selon les méthodes de test précitées, qui seront détaillées ultérieurement.

Selon un mode préféré d'exécution de l'invention, on utilise une composition parfumante contenant au moins 60% en poids ou plus d'ingrédients parfumants présentant chacun une activité bactéricide et/ou fongicide d'au moins 80% telle que mesurée par la ou les méthodes décrites ci-après et au moins 0,5% en poids d'un ingrédient actif sélectionné parmi les extraits de plantes ou les composés correspondant au principe actif de ces plantes cités plus haut.

Nous avons constaté que de meilleurs résultats étaient obtenus lorsqu'on utilisait des ingrédients parfumants répondant positivement à 100%, ou à un pourcentage très proche de cette valeur, à l'une quelconque des méthodes de test selon l'invention.

Ces ingrédients parfumants et les extraits de plantes, respectivement les composés correspondants, peuvent être ajoutés directement à des formulations classiques de base parfumante pour former un parfum renfermant des notes olfactives agréables. Le parfum ainsi formulé sera mélangé dans des concentrations comprises entre 0,1 et 20% en poids, par rapport au poids du produit auquel il sera incorporé. Des concentrations de l'ordre de 0,5 à 2% en parfum se sont révélées particulièrement avantageuses pour les applications en parfumerie fine envisagées selon l'invention.

Les ingrédients parfumants à action antimicrobienne, sont choisis dans des classes chimiques variées, comprenant par exemple des esters, des aldéhydes, des alcools, des éthers, des cétones, des acétals, des nitriles, des hydrocarbures terpéniques, des composés hétérocycliques azotés ou soufrés, ainsi que des huiles essentielles d'origine naturelle ou synthétiques. Le choix des ingrédients sera dicté par une réponse positive à 80% à l'un quelconque ou aux deux tests envisagés, ainsi que par l'effet olfactif recherché.

La composition parfumante de l'invention pourra contenir également d'autres ingrédients dont la contribution à la note olfactive finale sera purement hédonique. Typiquement elle contiendra aussi des ingrédients parfumants d'usage courant dont l'effet est éminemment olfactif et qui, à l'instar des ingrédients antimicrobiens susmentionnés, peuvent être choisis parmi les classes chimiques déjà citées plus haut. Le choix de ces ingrédients dépendra de la nature du produit que l'on désire parfumer, ainsi que, bien entendu du goût du parfumeur créateur.

Lors de l'un ou l'autre des tests, le pourcentage d'activité antimicrobienne de l'ingrédient parfumant est mesuré par rapport à son efficacité à réduire la flore microbienne avec laquelle il est en contact. Ainsi le résultat de l'activité bactéricide et/ou fongicide, effectuée dans les mêmes conditions pour les diverses substances odorantes choisies parmi les classes chimiques variées citées plus haut ou les huiles essentielles naturelles ou synthétiques, est analysé et répertorié selon le pourcentage d'activité constaté.

Par conséquent, un ingrédient parfumant, dès lors qu'il est capable de tuer la variété de microorganismes avec laquelle il est en contact, est microbicide et s'avère avantageux lorsqu'il montre une activité d'au moins 80% selon les tests envisagés.

Pour mesurer l'activité antimicrobienne d'un ingrédient parfumant, d'une composition parfumante définie selon l'invention ou d'un produit parfumé, on a procédé comme suit.

Quel que soit le test envisagé selon l'invention, on a réalisé en parallèle une culture du germe choisi sur des plaques de Petri dans les mêmes conditions de test, avec une solution témoin composée, en général, d'eau stérilisée ou d'une solution saline. Après la ou les périodes d'incubation envisagées selon le test choisi, on a mesuré la surface de la plaque exempte de bactéries et/ou de fongi pour le composé testé et pour la solution témoin. En effectuant un rapport de surface, on a défini une valeur relative de surface permettant de sélectionner les composés présentant une activité antimicrobienne d'au moins 80% suite à l'un de ces tests d'efficacité. Dans ces méthodes, on a utilisé les germes suivants : *Pityrosporum ovale, Staphylococcus aureus, Staphylococcus epidermidis, Escherichia coli, Pseudomonas aeruginosa, Candida albicans, Trychophyton mentagrophytes.*

La méthode de "test par diffusion de vapeurs" (VPT) permet de déterminer l'efficacité antimicrobienne des vapeurs soit d'un ingrédient parfumant, soit d'une composition parfumante, soit d'un article parfumé, sur une surface d'agar préalablement inoculée avec le microorganisme souhaité. Cette méthode se révèle particulièrement utile pour mesurer l'activité antimicrobienne de désodorisants ambiants.

Le "test par diffusion de vapeurs" est réalisé de la façon suivante.

Une boîte de Petri d'environ 20 mm de diamètre est placée au centre d'une boite de Petri plus large - 90 mm de diamètre -, de façon à ce que le gel nutritif chaud, versé dans la grande boite de Petri, entoure la petite boîte de Petri. Une fois la surface du gel nutritif solidifiée, celle-ci est infectée avec le germe désiré, et 1 ml du composé concentré à tester, est versé dans la petite boîte de Petri. On recouvre l'ensemble avec le couvercle de la grande boîte de Pétri.

Le gel nutritif est alors incubé à température ambiante ou à n'importe quelle température qui permettra la meilleur croissance possible des germes testés, pendant 72 heures. L'action antimicrobienne de l'ingrédient parfumant, de la composition parfumante ou du produit parfumé fini est définie selon le développement des germes après trois jours d'incubation. Si l'on constate un développement considérable de microorganismes, ceci nous indique que les vapeurs du composé ou composition testés ne sont pas actives selon le test. Si par contre on constate qu'aucune culture du germe n'a poussé, ou que pas plus de 20% de la surface de la plaque de Petri n'est recouverte par le germe, ceci nous indique une activité microbicide ou microbiostatique du composé ou composition envisagés.

Afin de déterminer l'activité spécifique telle que mesurée par le test, la petite boîte de Petri située au centre de la large est retirée à l'aide de pinces stériles, le couvercle est replacé et le gel nutritif ainsi dépourvu des vapeurs du composé testé est incubé pendant 72 heures dans les conditions optimales de croissance du germe choisi.

Après ces 72 heures, lorsqu'aucun développement de microbes n'est observé, nous pouvons en conclure que les vapeurs du composé testé utilisées précédemment avaient une bonne activité antimicrobienne, au moins 80% d'activité positive, et que par conséquent l'ingrédient parfumant, la composition parfumante ou le produit parfumé choisi a une action microbicide en tuant complètement les germes. Si, après ces 72 heures, un développement de microbes s'avère constaté, nous pouvons en conclure que les vapeurs du composé testé utilisées précédemment étaient seulement microbiostatiques, empêchant le développement des germes. Il convient de réaliser en parallèle une culture de germes dans des conditions favorables de croissance en absence de toutes traces de vapeurs du composé que l'on désire tester. En comparaison avec cette culture, seul l'ingrédient parfumant, la composition parfumante ou le produit parfumé choisi qui présente une activité microbiostatique d'au moins 80% selon le test est sélectionné.

La méthode "test sur surface agar enduite" permet de déterminer l'activité antimicrobienne de parfums ou de produits finis, parfumés ou pas, après lavage(s) de la surface testée.

On utilise une boîte de Petri standard de 90 mm de diamètre, disponible dans le commerce et portant déjà le gel nutritif. A l'aide d'une pipette automatique Gilson, on dépose 0,2 ml de bouillon de culture (déjà inoculé avec le germe voulu et incubé à température ambiante pendant 2 à 7 jours) sur la surface agar et on le répartit uniformément sur celle-ci à l'aide d'une spatule en verre. On incube pendant 3 heures à 37°C en prenant soin de laisser la boîte de Petri à moitié ouverte afin de permettre un léger assèchement du milieu et une bonne implantation des germes sur la surface. Puis 5 ml d'une concentration adéquate du produit ou agent à tester, sont déposés à la surface du gel. Par exemple, pour tester un shampoing, il convient de le diluer avec de l'eau stérilisée dans les proportions 1/6. Ceci correspondant approximativement à une concentration de shampoing appliquée sur une chevelure mouillée. Le produit à tester est laissé en contact 30 secondes avec la surface agar infectée, puis il est retiré et l'on rince celle-ci avec 10 ml d'eau stérilisée. On incube pendant au minimum 24 heures à température ambiante. Après cette première phase du test, on peut d'ores et déjà observer la surface du gel nutritif et constater s'il y a une évolution ou non quant à l'action antimicrobienne.

Un nouveau contact de 30 secondes entre le produit à tester et la surface agar est effectué dans les mêmes conditions que ci-dessus, suivi de l'étape de rinçage et d'incubation de 24 heures à température ambiante, l'évolution antimicrobienne est à nouveau constatée suite à cette seconde phase du test. Cette opération est renouvelée une troisième fois dans les mêmes conditions à l'exception du fait que l'on rince par 3 fois avec 10 ml d'eau stérilisée la surface du gel nutritif. Suivant l'application envisagée de l'invention, le composé testé est laissé en contact de 30 secondes à 5 minutes avec la surface agar infectée, puis la surface est rincée 3 fois avec 10 ml d'eau distillée. L'action microbicide ou microbiostatique peut être constatée après chaque phase du test et être répertoriée. Cette méthode permet de sélectionner les ingrédients actifs à au moins 80% selon le test et de tester l'efficacité de l'action antimicrobienne d'une composition parfumante ou d'un produit parfumé conçus selon l'invention, et plus particulièrement ceux qui présentent une activité microbicide dès la première ou seconde phase du test.

La méthode du spray direct, ou "MSD" a été décrite dans WO 93/25185 dont le contenu est indu ici par référence, notamment la description y faite de cette méthode et de son utilisation pour tester l'efficacité bactéricide ou antimicrobienne de préparations cosmétiques appliquées sur la peau, notamment des produits de type aérosol.

Selon cette méthode, grâce à. une valve aérosol doseuse, la composition désodorisante ou antiperspirante prête à l'usage, ou une solution correspondante des matières actives, est giclée directement sur la surface d'un gel nutritif préalablement infecté avec le germe souhaité. Celui-ci est alors incubé à 37°C pendant 24 ou 48 heures en vue de l'évaluation subséquente de bactéries sur la surface circulaire traitée. L'action antimicrobienne de la composition parfumée est ensuite définie par l'évaluation de la surface de la zone exemple de bactéries, par rapport à la surface totale circulaire traitée. Nous avons constaté que les compositions parfumées selon l'invention avaient une bonne activité antimicrobienne quand le rapport entre ces deux surfaces étaient d'environ 80% ou plus, lors du traitement du gel nutritif inoculé de germes avec ladite composition ou son principe actif.

La préparation, l'essai de plusieurs compositions parfumantes, a révélé que l'on arrive aux meilleurs résultats en appliquant les méthodes de test indiquées ci-dessus pour la sélection et la quantité d'ingrédients parfumants envisagés. Par exemple, lorsqu'une composition parfumante contient des ingrédients en quantité inférieure au minimum de 40%, il est improbable qu'elle ait une action antimicrobienne d'au moins 80% mesurée selon les méthodes de test de l'invention. Par conséquent, pour la préparation des meilleures compositions parfumantes, le choix des ingrédients parfumants en fonction de leur activité antimicrobienne mesurée selon l'un quelconque ou tous les tests de l'invention et la quantité d'ingrédients actifs envisagés, sont autant de facteurs importants à respecter pour obtenir les meilleurs résultats. Ainsi un parfum selon l'invention contiendra aussi bien des ingrédients parfumants d'usage courant choisis pour leur note olfactive particulière, que des ingrédients présentant à la fois une action antimicrobienne d'au moins 80% telle que mesurée selon la ou les méthodes de l'invention et un effet olfactif agréable, ou encore des ingrédients présentant principalement une activité antimicrobienne comme définie selon l'invention.

Suivant la nature des produits variés auxquels sera incorporée la composition parfumante, elle pourra être utilisée telle quelle, sous forme de solution, mélangée à un propulseur pour aérosol, ou encore mélangée à des ingrédients de nature variée couramment utilisés dans ces produits et illustrés dans les exemples présentés plus loin.

En effet, une composition parfumante selon l'invention a un emploi assez étendu et peut notamment être utilisée avantageusement pour parfumer des produits destinés à nettoyer et adoucir les textiles. Elle peut également être incorporée à des produits destinés à nettoyer des surfaces ménagères (sol, carrelage, faïence par exemple) ou à désinfecter et désodoriser l'air ambiant (déodorants ambiants de salles de bain, de toilettes, d'armoires par exemple), exerçant ainsi une action bactéricide et/ou fongicide sur les surfaces nettoyées ou par diffusion de vapeurs dans l'air ambiant.

La méthode ASCT se trouve être particulièrement appropriée pour tester des produits parfumés destinés à laver la peau ou les cheveux ou des surfaces variées dans l'industrie et le ménage. Elle se révèle parfaitement adaptée, par exemple, pour tester un shampoing parfumé destiné à des cheveux que l'on lave tous les jours et qui sont en contact avec le shampoing environ 30 secondes. Comme cité précédemment, nous avons pu établir que des ingrédients parfumants présentant une activité antimicrobienne d'au moins 80% telle que mesurée selon ladite méthode, pouvaient être avantageusement utilisés pour préparer des compositions parfumantes conformes à l'invention, à savoir des compositions contenant au moins 40% en poids d'ingrédients actifs et ayant une activité antimicrobienne efficace. Le tableau 1 ci-dessous présente, en effet, les résultats obtenus quant à l'activité antimicrobienne telle que mesurée par la méthode ASCT de l'invention à l'encontre du micro-organisme *Pityrosporum ovale -* champignon associé à la formation de pellicules sur le cuir chevelu- d'ingrédients parfumants individuels testés dans un shampoing et énumérés ici en une liste non exhaustive. Par shampoing, on entend ici une base shampoing de type courant quelconque. A titre d'exemple, on peut utiliser une base telle que celle préparée par mélange des ingrédients suivants :

| Ingrédients | Parties en poids |
|---|---|
| Texapon® N SO [1] | 25,0 |
| Eau déminéralisée | 58,60 |
| Kathon CG[2] | 0,10 |
| Miracare® 2MCA/P [3] | 15,00 |
| Comperlan® KD [4] | 1,30 |
| | 100,00 |

1) sulfate de sodium laureth ; origine : Henkel, Allemagne

2) méthylchloroisothiazolinone (et) méthylisothiazolinone ; origine : Rohm & Haas, USA

3) disodium cocoamphodiacétate (et) sulfate de sodium lauryle (et) hexylène glycol ; origine : Rhône-Poulenc, France

4) cocamide DEA ; origine : Henkel, Allemagne

Il est cependant clair que n'importe quelle autre base shampoing pourrait être utilisée. Il convient de noter que les initiales BC et BS présentes dans ce tableau correspondent respectivement aux abréviations des termes bactéricide et bactériostatique explicités précédemment lors de la description des tests de l'invention.

## Tableau 1

| Action contre Pityrosporum ovale | Test sur surface agar enduite (ASCT) | | | | |
|---|---|---|---|---|---|
| Ingrédients parfumants | Court temps de contact 3 x 30 secondes en 3 jours | | | | |
| | 1ère application | 2ème application 24 h | | 3ème application 48 h | |
| Concentration dans le shampoing | 0,5% | 0,5% | 0,1% | 0,5% | 0,1% |
| Acétate de benzyle | x | BC | BC | - | - |
| Acétate de cinnamyle | x | - | BS | - | BC |
| Acétate de phényléthyle | x | BC | BS | - | BC |
| Acétate de phénylpropyle | x | BS | x | BC | BC |
| Acétate de prényle | x | BS | - | BC | - |
| Acétate de styrallyle | x | BC | x | - | BS |
| Acétophénone | BC | - | BC | - | - |
| Alcool cinnamique ord. | x | BC | x | - | BS |
| Aldéhyde anisique spécial redist. | x | BC | x | - | BC |
| Aldéhyde benzoïque | x | x | x | BC | BC |
| Aldéhyde cinnamique | x | BC | x | - | BS |
| Aldéhyde méthylcinnamique | x | BC | BC | - | - |
| Anisate de méthyle | BC | - | BC | - | - |

| | | | | | |
|---|---|---|---|---|---|
| Anthranilate de méthyle dist. | x | BC | BS | - | BC |
| Benzoate d'éthyle | x | BC | BS | - | BC |
| Benzoate de méthyle | x | BC | BS | - | BC |
| Benzylacétone | x | - | BC | - | - |
| 2,4-Diméthyl-3-cyclohexène-1-carbaldéhyde [2] | x | x | - | BC | - |
| 4-Cyclohexyl-2-méthyl-2-butanol [2] | x | x | - | BC | - |
| Coumarine | BC | - | BC | - | - |
| γ-Décalactone | x | BC | x | - | BC |
| Dihydromyrcénol | x | x | - | BC | - |
| Essence de bergamote | x | BC | - | - | - |
| Essence de bois de rose du Brésil | x | BC | x | - | BS |
| Essence de cannelle de Ceylan synth. | x | BC | BC | - | - |
| Essence de cannelle de Ceylan | x | BC | BS | - | BC |
| Essence de cannelle de Chine | BC | - | BS | - | BC |
| Essence de cèdre ord. | x | x | - | BC | - |
| Essence de coriandre | x | BC | x | - | BC |
| Essence d'eucalyptus | x | x | x | BC | BS |
| Essence de feuillage vert | x | BC | x | - | BS |
| Essence de feuilles de cannelier | x | BC | x | - | BS |
| Essence de girofle | x | BC | x | - | BC |
| Essence de jasmin | x | x | x | BC | BS |
| Essence de thuya | x | x | - | BC | - |
| Eugénol | x | BS | x | BC | BS |
| Florex ® [2] | x | BS | x | BC | BS |
| Formiate de benzyle | x | BC | x | - | BC |
| Géraniol brut | x | BC | x | - | BS |
| Hédione ® [2] | x | x | - | BC | - |
| Indolarome ® [1] | x | BC | BS | - | BS |
| Linalol | x | x | - | BC | - |
| Menthone purifiée | x | BS | - | BC | - |
| Méthylacétophénone | x | BC | BC | - | - |
| p-Méthylphénol | x | x | x | BC | BC |
| 4-Nonanolide | x | BC | x | - | BC |
| Phénylhexanol | x | BC | x | - | BS |
| Salicylate de méthyle | x | BC | BS | - | BC |
| γ-Undecalactone | x | x | - | BC | - |

1) International Flavors and Fragrances, USA

2)  Firmenich SA, Genève, Suisse

BC = bactéricide

BS = bactériostatique

x = sans activité antimicrobienne

- = non testé

Comme le montrent les résultats décrits ci-dessus, la méthode ASCT a permis d'identifier plusieurs ingrédients parfumants selon l'invention pouvant être utilisés pour élaborer une composition parfumante particulièrement active à l'encontre du *Pityrosporum ovale* et destinée à être employée dans un shampoing comme cité précédemment.

A titre d'ingrédients parfumants présentant une activité antimicrobienne d'au moins 80% telle que mesurée par la ou les méthodes de test de l'invention, on peut également citer l'essence d'absinthe, l'essence d'armoise, l'essence de laurier, l'essence de bois de rose du Brésil, l'acétate d'hexyle, l'éthyl vinyl cétone, l'oxyde de rose, l'$\alpha$-pinène, l'acétate d'ormenthyle, l'acétate de cyclohexyle, l'acétophenone, l'aldéhyde phényl- propionique, l'essence de bergamote synthétique et l'essence de cannelle de Chine.

L'invention sera décrite plus en détail à l'aide des exemples suivants.

**Manières de réaliser l'invention**

Exemple 1

Composition parfumante

On a préparé une composition parfumante à activité antimicrobienne, par mélange des ingrédients suivants :

| Ingrédients | Parties en poids |
| --- | --- |
| Acétate d'hexyle [1)2)] | 5,0 |
| Acétate d'isobomyle [1)] | 8,0 |
| Acétate de linalyle [1)] | 9,2 |
| Ambrox® [1)3)4)] | 0,3 |
| Essence de bergamote [2)] | 18,0 |
| Camphre [1)2)] | 2,5 |
| Essence de cèdre ord. [1)] | 8,5 |
| Propionate de tricyclo [5.2.1.0$^{2.6}$]dec-3-én-8-yle [1)3)] | 3,5 |
| Coumarine [1)] | 4,0 |
| Dihydromyrcénol [1)2)] | 14,0 |
| Dihydroterpinéol [1)2)] | 12,5 |
| Diphényloxyde [1)] | 1,5 |
| 3-p-Menthanone [1)2)] | 4,0 |
| Néroloxyde [1)2)] | 0,5 |
| Tétralinol [1)2)] | 6,5 |
| 2,4-Diméthyl-3 -cyclohexène- 1 -carbaldéhyde [1)2)3)] | 2,0 |
| Total | 100,0 |

1) ingrédient parfumant présentant une activité antimicrobienne d'au moins 80% telle que mesurée par la méthode "test sur surface agar enduite" (ASCT)

2) ingrédient parfumant présentant une activité antimicrobienne d'au moins 80% telle que mesurée par la méthode "test par diffusion de vapeurs" (VPT)

3) origine : Firmenich SA, Genève, Suisse

4) 8,12-époxy-13,14,15,16-tétranorlabdane

Les ingrédients susmentionnés ont été mélangés selon les techniques courantes connues de l'homme du métier.

Cette composition parfumante a montré une activité antimicrobienne d'au moins 80% telle que mesurée par la méthode ASCT ou par la méthode VPT et peut être utilisée pour fabriquer une composition parfumante de l'invention, avec d'autres ingrédients tels que décrits.

Exemple 2

Composition parfumante

Une composition parfumante de type jasmin ayant une activité antimicrobienne à l'encontre du *Pityrosporum ovale* et destinée à être employée dans un shampoing, a été préparée par mélange des ingrédients suivants :

| Ingrédients | Parties en poids |
|---|---|
| Acétate de benzyle [1] | 14,0 |
| Acétate de linalyle | 5,0 |
| Alcool cinnamique [1] | 3,0 |
| Alcool phényléthylique | 4,0 |
| Aldéhyde $\alpha$-amylcinnamique | 6,0 |
| Anthranilate de méthyle dist. [1] | 1,5 |
| Benzoate de benzyle | 18,0 |
| Eugénol [1] | 1,0 |
| Florex® [1,2] | 10,0 |
| Géraniol brut [1] | 5,0 |
| Essence de girofle [1] | 0,5 |
| Hédione® [1,3] | 20,0 |
| Hydroxycitronellal | 2,0 |
| Indolarome® [1,4] | 1,5 |
| Jasmonate de méthyle | 1,0 |
| Linalol [1] | 7,5 |
| Total | 100,0 |

1) ingrédient parfumant présentant une activité antimicrobienne d'au moins 80% telle que mesurée par la méthode "test sur surface agar enduite" (ASCT)

2) 6 et 7-éthylidèneoctahydro-5,8-méthano-2H-1-benzopyran-2-one ; origine : Firmenich SA, Genève, Suisse

3) 3-oxo-2-pentyl-cyclopentaneacétate de méthyle ; origine : Firmenich SA, Genève, Suisse

4) 4,4a,5,9b-tétrahydro-indeno[1,2-D]-1,3-dioxine ; origine : International Flavors & Fragrances, USA

Les ingrédients susmentionnés ont été mélangés selon les techniques courantes connues de l'homme du métier.

Cette composition parfumante de type jasmin a montré une activité antimicrobienne d'au moins 80% telle que mesurée par la méthode ASCT contre le *Pityrosporum ovale.* Elle peut être utilisée pour fabriquer une composition parfumante de l'invention, avec d'autres ingrédients tels que décrits.

Exemple 3

Shampoing antipelliculaire parfumé testé par la méthode "test sur surface agar enduite"

Un shampoing antipelliculaire parfumé à action antimicrobienne, destiné à être employé lors de lavages fréquents, a été préparé à l'aide des ingrédients suivants :

| Ingrédients | Shampoing I | Shampoing II |
|---|---|---|
| | Parties en poids | Parties en poids |
| **A** Texapon® NSO [1] | 25,00 | 25,00 |

1) sulfate de sodium laureth ; origine : Henkel, Allemagne

(suite)

| Ingrédients | Shampoing I | Shampoing II |
|---|---|---|
|  | Parties en poids | Parties en poids |
| Octopirox ® 2) | 0,60 | 0,60 |
| **B** Eau déminéralisée | 57,10 | 57,90 |
| Acide fumarique | 0,20 | 0,20 |
| **C** Miranol® 2MCA mod.3) | 15,00 | 15,00 |
| **D** Comperlan® KD 4) | 1,30 | 1,30 |
| **E** Parfum 5) | 0,80 | ----- |
| Total | 100,00 | 100,00 |

2) piroctone olamine ; origine : Hoechst AG, Allemagne

3) disodium cocoamphodiacetate (et) sulfate de sodium lauryle (et) hexylène glycol ; origine : Rhône-Poulenc, France

4) cocamide DEA ; origine : Henkel, Allemagne

5) voir exemple 1 ou 2, ou encore Activa 147.121 X ; origine : Firmenich SA, Genève, Suisse

On a dissous l'Octopirox® dans le Texapon® NSO. On a préparé la partie B, on l'a versée dans la partie A et on a mélangé. On a ajouté la partie C, on a mélangé à nouveau. On a ajouté la partie D, on a mélangé et enfin on a ajouté le parfum E.

L'Octopirox® est un fongicide, utilisé couramment en concentration de 0,5 - 1,0 % dans les préparations antipelliculaires, ayant une bonne activité contre le *Pityrosporum ovale* [noté *P. ovale* (IHEM 3967), champignon associé à la formation de pellicules sur le cuir chevelu]. On a constaté que la croissance de *P. ovale,* lors de l'utilisation d'un shampoing avec Octopirox®, est diminuée de 60% (mesurée selon la présente méthode de test) en comparaison avec un shampoing sans Octopirox®.

D'autre part, la comparaison des shampoings I et II décrits ci-dessus a montré que la combinaison de l'Octopyrox® avec le parfum et l'acide fumarique selon l'invention présente une activité antimicrobienne nettement plus prononcée. En effet, lors du test effectué avec le shampoing I, on a observé une inhibition complète de la croissance de *P. ovale.* Le produit parfumé possédait une activité antimicrobienne moyenne de 90%, valeur relative de surface, mesurée selon la méthode "test sur surface agar enduite".

Exemple 4

Shampoing antipelliculaire parfumé testé par la méthode "test sur surface agar enduite"

Le Zinc-Pyrion ® 1) est un fongicide très puissant agissant aussi contre *P. ovale* et utilisé à 0,5 - 1,0% surtout dans les shampoings antipelliculaires opaques.

La formule suivante a été testée :

| Ingrédients | | Parties en poids |
|---|---|---|
| **A** | Eau déminéralisée | 46,80 |
|  | Zinc-Pyrion ® | 0,60 |
|  | Gelwhite USP 2) | 1,00 |
| **B** | Eau déminéralisée | 20,15 |
|  | Chlorure de sodium | 2,00 |
|  | Acide fumarique | 0,05 |

1) Zinc-Pyrion ® ; origine : Pyrion-Chemie, Allemagne 2) origine : Euroclay, Hollande

(suite)

| Ingrédients | | Parties en poids |
|---|---|---|
| | Nutrilan ® L [3] | 1,00 |
| **C** | Texapon ® NSO [4] | 50,00 |
| | Viscofil. Blau BL [5] solution à 0,5% | 0,40 |
| | Butylene glycol | 2,00 |
| | Comperlan® KD[6] | 2,00 |
| **D** | Parfum [7] | 0,80 |
| | Total | 100,00 |

3) collagène hydrolysé ; origine : Henkel, Allemagne

4) sulfate de sodium laureth ; origine : Henkel, Allemagne

5) origine : Sandoz, Suisse

6) cocamide DEA ; origine : Henkel, Allemagne

7) voir exemple 1 ou 2, ou encore Activa 147.121 X ; origine : Firmenich SA, Genève, Suisse

Sous agitation rapide à la turbine, on a dispersé le Gelwhite dans l'eau. On a ajouté le Zinc-Pyrion, toujours sous agitation.

On a préparé la partie B en dissolvant tous les composés dans l'eau.

On a préparé la partie C en mélangeant bien de façon à avoir une préparation homogène.

On a versé la partie A dans la partie C et on a mélangé bien à l'aide de la turbine. On a versé la partie B dans la partie A + C. On a mélangé bien jusqu'à ce que le shampoing soit bien homogène.

La croissance de *P. ovale* est inhibée complètement après les 3 lavages consécutifs avec ce shampoing, la surface de la plaque reste propre. L'activité antimicrobienne du shampoing parfumé est supérieure à 80% telle que mesurée par la méthode ASCT.

<u>Exemple 5</u>

<u>Gel douche et mousse de bain</u>

La composition suivante a été testée selon la méthode "test sur surface agar enduite", contre l'action microbienne du *Staphylococcuc aureus,* avec et en l'absence de parfum.

| Ingrédients | Parties en poids |
|---|---|
| Eau déminéralisée | 46,30 |
| Kathon CG [1] | 0,10 |
| Acide fumarique | 0,10 |
| Chlorure de sodium | 2,00 |
| Euperlan ® PK 771 [2] | 5,00 |
| Texapon ® T42 [3] | 20,00 |
| Texapon ® NSO [4] | 20,00 |
| Genaminox ® KC [5] | 5,00 |
| Parfum [6] | 1,50 |
| Total | 100,00 |

1) méthylchloroisothiazolinone (et) méthylisothiazolinonc ; origine : Rohm & Haas, USA

2) distéarate de glycol (et) sulfate de sodium laureth (et) MEA cocamide (et) laureth-10 ; origine : Henkel, Allemagne

3) TEA lauryl sulfate ; origine : Henkel, Allemagne

4) sodium laureth sulfate ; origine : Henkel, Allemagne

5) oxide de cocamide ; origine : Hoechst AG, Allemagne

6) composition selon l'exemple 1 ou Manzana 147.038 B (1,0%) et essence de cèdre Texas (0,5%) ; origine : Firmenich SA, Genève, Suisse

On a mélangé tous les ingrédients dans la séquence indiquée, puis on a ajouté le parfum.

Sans parfum, la plaque de Petri est entièrement recouverte d'une couche du germe testé, le *Staphylococcus aureus.* Suite au test effectué en présence de parfum, la surface de la plaque de Petri est pratiquement propre. Seules quelques petites colonies du germe ont pu être observées. Ainsi la composition ci-dessus de gel douche et mousse de bain présente une activité antimicrobienne, contre le *Staphylococcus aureus,* de 85% telle que mesurée selon la méthode "test sur surface agar enduite".

Exemple 6

Gel de douche ou bain

La composition suivante a été testée selon la méthode "test sur surface agar enduite", contre l'action antimicrobienne du *Pseudomonas aeruginosa,* avec et en l'absence du parfum.

| Ingrédients | Parties en poids |
|---|---|
| Eau déminéralisée | 48,55 |
| Acide fumarique | 0,75 |
| Extrait de fumeterre | 0,10 |
| Acide citrique | 0,05 |
| EDTA B (poudre)[1] | 0,05 |
| Luviquat Mono CP [2] | 1,00 |
| CAE [3] | 0,40 |
| Texapon NSO 15 [4] | 35,00 |
| Tego-Betain L7 [5] | 5,00 |
| Plantacare 2000 [6] | 4,00 |
| Kathon CG [7] | 0,10 |
| Cetiol HE [8] | 2,00 |
| Parfum [9] | 1,00 |
| Chlorure de Sodium | 2,00 |
| Total | 100,00 |

1) origine : BASF AG, Allemagne

2) hydroxyéthyl cétyldimonium phosphate ; origine : BASF AG, Allemagne

3) PCA éthyl cocoyl arginate ; origine : Ajinomoto, Japon

4) sodium laureth sulfate ; origine : Henkel, Allemagne

5) cocoamidopropyl bétaine ; origine : Henkel, Allemagne

6) décyl polyglucose ; origine : Henkel, Allemagne

7) mélange de méthylchloroisothiazolinone et de méthylisothiazolinone ; origine : Rohm and Haas, USA

8) PEG-7 glycéryl cocoate ; origine : Henkel, Allemagne

9) composition selon les exemples 1 ou 2

On a mélangé tous les ingrédients dans la séquence indiquée, puis on a ajouté le parfum.
Sans parfum, la plaque de Petri est entièrement recouverte d'une couche du germe testé, le *Pseudomonas aeruginosa.* Suite au test effectué en présence de parfum, la surface de la plaque de Petri est pratiquement propre. Seules quelques petites colonies du germe ont pu être observées. Ainsi la composition ci-dessus de gel douche présente une activité antimicrobienne, contre le *Pseudomonas aeruginosa,* mesurée selon la méthode "test sur surface agar enduite".

Exemple 7

Désodorisant ambiant

Un désodorisant ambiant à paroi de diffusion polymérique, ayant une surface de diffusion de 80 cm$^2$, contenant 5 g de l'un des parfums cités aux exemples 1 et 2 ou encore un parfum de type Cherry 20.599 (origine : Firmenich SA, Genève, Suisse) et 0,5 g de fumarate de diéthyle, est placé dans un fût en inox de 200 litres. A côté, sur le fond, on a placé quatre boîtes de Petri, chacune préalablement inoculée avec un germe différent. Les germes utilisés étaient les suivants *: Staphylococcus aureus, Escherichia coli, Pseudomonas aeruginosa, Candida albicans.* Le fût a été fermé et laissé à température ambiante pendant 3 jours. On a effectué en parallèle la même expérience mais sans désodorisant ambiant.

On a constaté, après 3 jours d'incubation, que le fût contenant le désodorisant ambiant avait une odeur agréable et qu'aucune croissance de germes sur les plaques de Petri n'était observée.

Alors que le fût sans désodorisant ambiant possédait une forte odeur de décomposition, les plaques révélant à la surface de l'agar une nette croissance de bactéries.

Exemple 8

Savon désodorisant de type synthétique

| Ingrédients | Parties en poids |
|---|---|
| Tensianol ® Scils [1] | 92,50 |
| Acide fumarique | 1,50 |
| Surfadone ® LP-300 | 2,00 |
| Cosmacol ® EL1 | 2,50 |
| Parfum [2] | 1,50 |
| Total | 100,00 |

1) Cocoisethionate de sodium ; origine : Manro Chemicals, Grande-Bretagne

2) composition selon l'exemple 1 ou encore Astéria 147.037 B (1,5%) et essence de cèdre Texas (0,5%) ; origine : Firmenich SA, Genève, Suisse

Ce mélange a été extrudé et pressé en morceaux de savon avec l'équipement usuel.

Le savon résultant a été dissous à 5% dans de l'eau. Lors du test ASCT effectué avec le savon non parfumé, on a constaté, suite à une incubation de 72 heures à température ambiante, que toute la surface de la plaque de Petri était recouverte d'une couche de bactéries du genre *Staphilococcus epidermidis.* Par contre, lors du test effectué avec le savon parfumé, on a constaté que seules quelques colonies du germe étaient réparties à la surface de la plaque de Petri. Le savon parfumé montrait une activité antimicrobienne de 80% telle que mesurée par la méthode ASCT.

Exemple 9

Adoucissant textile

Un adoucissant textile a été préparé à partir des ingrédients suivants :

| Ingrédients | Parties en poids |
|---|---|
| Praepagen ®WK [1] | 6,5 |
| Formaline à 40% | 0,2 |
| Eau déminéralisée | 91,8 |
| Colorant [2] | 0,1 |
| Acide fumarique | 0,5 |
| Fumarate de digéranyle [3] | 0,2 |
| Parfum [4] | 0,7 |
| Total | 100,00 |

1) poudre de chlorure distéaryldiméthylammonium ; origine : Hoechst AG, Allemagne

2) Bleu de Colanyle AR / solution à 10%

3) origine : Firmenich SA, Genève, Suisse

4) composition selon les exemples 1 ou 2

On a chauffé l'eau à 60°C, ajouté le Praepagen ® WK et mélangé jusqu'à dispersion complète. On a laissé refroidir à température ambiante en continuant à ajouter le mélange. Puis on a ajouté le colorant, l'acide fumarique, le fumarate de digéranyle, le formaline et le parfum.

Si la viscosité du mélange final n'est pas suffisamment haute, celle-ci peut être augmentée en ajoutant de petites quantités de NaCl. Par contre, si le mélange est trop épais, la viscosité peut être baissée en ajoutant 2,5% d'isopropanol. On a obtenu ainsi un adoucissant textile présentant une activité antimicrobienne.

Exemple 10

Déodorant corporel antimicrobien

Un déodorant a été préparé à partir des ingrédients suivants :

| | Ingrédients | Parties en poids |
|---|---|---|
| **A** | Citricidal®[1] | 0,8 |
| **B** | Cremophor® RH 40 [2] | 2,0 |
| | Dipropylène glycol | 14,0 |
| | Ethanol 95% | 18,5 |
| | Eau déminéralisée | 61,6 |
| | Acide fumarique | 0,5 |
| **C** | Phospholipide PTC | 0,6 |
| | Softigen ® 701 | 0,5 |
| **D** | Parfum [3] | 1,5 |
| | Total | 100,00 |

1) Extrait de pépin de pamplemousse; origine : BioChem, USA

2) PEG-40 huile de ricin hydrogénée ; origine : BASF, Allemagne

3) Composition selon les Exemples 1 ou 2

Les ingrédients ont été mélangés selon la séquence indiquée dans la formule ci-dessus. Le déodorant obtenu présentait une activité antimicrobienne.

Exemple 11

Bloc WC antimicrobien

Un bloc WC à action antimicrobienne a été préparé à partir des ingrédients suivants :

| Ingrédients | Parties en poids |
|---|---|
| Marlon ® A 390 [1] | 40,0 |
| Produit AAS 90 [2] | 30,0 |
| PEG 150 | 8,0 |
| Acide fumarique | 2,0 |
| Sulfate de sodium | 15,0 |
| Parfum [3] | 5,0 |
| Total | 100,00 |

1) Dodécylbenzènesulfonate de sodium ; origine : Hüls

2) Isopropylamine dodécylbenzènesulfonate ; origine : Henkel

3) Composition selon les Exemples 1 ou 2

On a bien mélangé les ingrédients dans l'ordre indiqué. Le produit ainsi obtenu a été extrudé ou pressé afin d'obtenir un bloc WC présentant une activité antimicrobienne.

Exemple 12

Nettoyant désodorisant pour WC

On a préparé un nettoyant pour WC à partir des ingrédients suivants :

| Ingrédients | Parties en poids |
|---|---|
| Eau déminéralisée | 77,750 |
| Acide fumarique | 1,000 |
| Ethanol | 3,000 |
| Parfum | 5,000 |
| Kelzan ® ST[1] | 1,250 |
| Dobanol ® 91-8 [2] | 10,000 |
| Empilan ® CDE [3] | 2,000 |
| Total | 100,00 |

1) Gomme de Xanthan ; origine : Monsanto

2) Ethoxylate à 8 moles d'oxyde d'éthylène basé sur des alcools $C_9$, $C_{11}$, et $C_{11}$; origine : Shell

3) Cocoamide DEA ; origine : Marchon, Grande-Bretagne

On a ajouté le Kelzan® ST à l'eau déminéralisée sous agitation modérée, puis on a laissé hydrater le produit pendant 20 minutes. On a ensuite mélangé le parfum, le Dobanol® 91-8, l'Empilan® et l'éthanol jusqu'à l'obtention d'une solution claire. Cette dernière a été ajoutée lentement au gel obtenu à partir de Kelzan ®, sous agitation modérée, jusqu'à la restructuration du gel. Après addition de l'acide fumarique, on a obtenu un gel qui se prête à l'utilisation en tant que nettoyant WC, présentant une action antimicrobienne de 100% mesurée selon le test ASCT.

Exemple 13

Disque en plastique désodorisant antimicrobien pour lave-vaisselle

Un disque pour lave-vaisselle sous forme de disque plastique a été préparé à partir des ingrédients suivants :

| Ingrédients | Parties en poids |
|---|---|
| Résine absorbante à base de Pebax® [1] ou d'éthylène vinylacétate | 80,0 |
| Parfum [2] | 15,0 |
| Fumarate de diéthyle | 5,0 |
| Total | 100,00 |

1) Polyaminoéther ; origine : ATO Chimie, France

2) Composition selon les Exemples 1 ou 2

On a mélangé les ingrédients identifiés ci-dessus et on les a mis dans un moule. On a ainsi obtenu un disque désodorisant présentant une activité antimicrobienne.

Exemple 14

Produit liquide pour lave-vaisselle

On a préparé un produit pour lave-vaisselle de type standard, à partir des ingrédients suivants :

| Ingrédients | Parties en poids |
|---|---|
| Marlon PS 65 [1] | 49,00 |
| Marlinat 242/70 [2] | 11,50 |
| Marlamid DF 1218 [3] | 3,00 |
| Eau déminéralisée | 32,00 |
| Ethanol à 96% volume | 4,00 |

1) $C_{13}$-$C_{17}$ alkane sulfonate de sodium ; origine : Hüls, Allemagne

2) Laureth sulfate de sodium ; origine : Hüls, Allemagne

3) Cocoamide DEA ; origine : Hüls, Allemagne

(suite)

| Ingrédients | Parties en poids |
|---|---|
| Parfum [4] | 0,50 |
| Total | $\overline{100,00}$ |

4) Composition selon les exemples 1 ou 2, ou le Nicosia 115.210 ou l'Orange 214.215 ; origine : Firmenich SA, Genève, Suisse

On a obtenu un lave-vaisselle liquide, en mélangeant les ingrédients ci-dessus. On a ensuite préparé un échantillon auquel on a ajouté 0,15% en poids du Triclosan et un autre échantillon auquel on a ajouté 0,05% en poids du Triclosan et 0,4% en poids de l'acide fumarique. Les deux échantillons ont été testés à leur activité antimicrobienne contre le *Pseudomonas aeruginosa,* selon la méthode "test sur surface agar enduite". On a pu observer que, en utilisant l'échantillon contenant la combinaison Triclosan plus acide fumarique, seulement quelques bactéries avaient survécu sur la surface de la plaque de Petri, alors qu'avec l'échantillon ne contenant pas d'acide fumarique, une grande partie de la surface était couverte par des bactéries.

Exemple 15

Produit d'entretien

Un produit d'entretien a été préparé à partir des ingrédients suivants :

| Ingrédients | Parties en poids |
|---|---|
| Eau déminéralisée | 74,50 |
| Carbonate de sodium | 3,00 |
| Citrate de sodium | 2,00 |
| Cumolsulphonate de sodium | 7,00 |
| Marlon (R) A 375 [1] | 10,00 |
| Tergitol 15-S-9 surfactant [2] | 3,00 |
| Parfum [3] | 0,50 |
| Total | $\overline{100,00}$ |

1 ) Dodécylbenzènesulfonate de sodium ; origine : Hüls, Allemagne

2) $C_{11}$-$C_{15}$ Pareth-9 ; origine : Union Carbide, USA

3) Composition selon les exemples 1 ou 2, ou le Nicosia 115.210 ou l'Orange 214.215 ; origine : Firmenich SA, Genève, Suisse

En mélangeant les ingrédients ci-dessus, on a obtenu un produit d'entretien du type standard. On a ensuite préparé un échantillon auquel on a ajouté 0,05% en poids du Triclosan et 0,40% en poids de l'acide fumarique et un autre échantillon auquel on a ajouté 0,15% en poids du Triclosan. L'activité antimicrobienne de ces deux échantillons a été déterminée, en utilisant le "test sur surface agar enduite", contre le *Pseudomonas aeruginosa.* On a pu observer que l'activité antimocrobienne de l'échantillon contenant l'acide fumarique plus le Triclosan était nettement plus prononcée que l'activité de l'échantillon contenant seulement le Triclosan.

**Revendications**

**1.**   Composition parfumante antimicrobienne, **caractérisée en ce que** ladite composition comprend

-   au moins 40% en poids d'ingrédients parfumants ayant chacun une activité antimicrobienne d'au moins 80% telle que mesurée par la méthode du « test sur surface agar enduite » (ASCT), par la méthode du « test par diffusion de vapeurs » (VPT) ou par la « méthode du spray direct » (MSD) ; et
-   au moins 0,1% d'un ingrédient actif sélectionné parmi un extrait de pépin de pamplemousse, un extrait de fumeterre, l'acide fumarique ou un ester de l'acide fumarique ou lactique.

**2.**   Composition selon la revendication 1, **caractérisée en ce que** ladite composition comprend un agent antimicrobien choisi parmi le Triclocarban et le Triclosan.

**3.** Composition selon la revendication 1 ou 2, **caractérisée en ce que** l'ester de l'acide fumarique est le fumarate de diéthyle ou le fumarate de digéranyle.

**4.** Composition selon l'une des revendications 1 à 3, **caractérisée en ce que** ladite composition comprend un agent tensioactif de nature anionique, cationique, nonionique ou amphotérique ou un phospholipide.

**5.** Composition selon la revendication 4, **caractérisée en ce que** l'agent tensioactif est un phospholipide ou un agent tensioactif quaternaire.

**6.** Composition selon l'une des revendications 1 à 5, **caractérisée en ce qu'**elle comprend un émollient d'usage courant en parfumerie ou cosmétique.

**7.** Composition selon la revendication 6, **caractérisée en ce que** l'émollient est un ester de l'acide lactique ou fumarique.

**8.** Composition selon l'une des revendications 1 à 7, **caractérisée en ce qu'**elle contient au moins 60% en poids d'ingrédients parfumants ayant chacun une activité antimicrobienne d'au moins 80% telle que mesurée par la méthode « ASCT » ou par la méthode « VPT » et au moins 0,1% en poids d'un ingrédient actif tel que spécifié à la revendication 1.

**9.** Composition selon la revendication 8, **caractérisée en ce que** chaque ingrédient parfumant présente une activité Anti-microbienne de 100% telle que mesurée par la méthode « ASCT » ou par la méthode « VPT ».

**10.** Article parfumé contenant une composition parfumante selon l'une des revendications 1 à 9.

**11.** A titre d'article parfumé selon la revendication 10, un savon, un gel de douche ou de bain, un shampooing ou autre produit d'hygiène capillaire, une préparation cosmétique, un déodorant ou antiperspirant corporel, un désodorisant d'air ambiant, un détergent ou adoucissant textile ou un produit d'entretien d'usage domestique ou industriel.

**12.** Utilisation d'une composition selon l'une des revendications 1 à 10 pour conférer une activité Anti-microbienne ou renforcer l'activité Anti-microbienne d'un article destiné aux soins corporels ou capillaires, ou d'un produit fonctionnel, **caractérisée en ce qu'**on ajoute à cet article ou produit une composition parfumante selon l'une des revendications 1 à 10.

**Patentansprüche**

**1.** Antimikrobielle Duftzusammensetzung, **dadurch gekennzeichnet, daß** die Zusammensetzung umfaßt:

- wenigstens 40 Gew.-% Duftbestandteile, die jeweils eine antimikrobielle Aktivität von wenigstens 80 % aufweisen, wie gemessen nach der Methode des "Tests auf bestrichener Agaroberfäche" (ASCT), nach der Methode des "Tests mittels Dampfdiffusion" (VPT) oder nach der "Methode des direkten Besprühens" (MSD); und

- wenigstens 0,1 % eines Wirkstoffs, ausgewählt aus einem Grapefruitkernextrakt, einem Erdrauchextrakt, Fumarsäure oder einem Fumarsäure- oder Milchsäureester.

**2.** Zusammensetzung nach Anspruch 1, **dadurch gekennzeichnet, daß** die Zusammensetzung ein antimikrobielles Mittel enthält, das ausgewählt ist aus Triclocarban und Triclosan.

**3.** Zusammensetzung nach Anspruch 1 oder 2, **dadurch gekennzeichnet, daß** der Fumarsäureester das Diethylfumarat oder das Digeranylfumarat ist.

**4.** Zusammensetzung nach einem der Ansprüche 1 bis 3, **dadurch gekennzeichnet, daß** die Zusammensetzung ein Tensid anionischer, kationischer, nichtionischer oder amphoterer Natur oder ein Phospholipid enthält.

**5.** Zusammensetzung nach Anspruch 4, **dadurch gekennzeichnet, daß** das Tensid ein Phospholipid oder ein quartäres Tensid ist.

**6.** Zusammensetzung nach einem der Ansprüche 1 bis 5, **dadurch gekennzeichnet, daß** sie ein in der Parfümerie oder Kosmetik übliches Emolliens umfaßt.

**7.** Zusammensetzung nach Anspruch 6, **dadurch gekennzeichnet, daß** das Emolliens ein Milchsäure- oder Fumarsäureester ist.

**8.** Zusammensetzung nach einem der Ansprüche 1 bis 7, **dadurch gekennzeichnet, daß** sie wenigstens 60 Gew.-% Duftbestandteile, die jeweils eine antimikrobielle Aktivität von wenigstens 80 % aufweisen, wie gemessen nach der "ASCT"-Methode oder nach der "VPT"-Methode, und wenigstens 0,1 Gew.-% eines wie in Anspruch 1 spezifizierten Wirkstoffs enthält.

**9.** Zusammensetzung nach Anspruch 8, **dadurch gekennzeichnet, daß** jeder Duftbestandteil eine antimikrobielle Aktivität von 100 % aufweist, wie gemessen nach der "ASCT"-Methode oder nach der "VPT"-Methode.

**10.** Parfümierter Artikel, enthaltend eine Duftzusammensetzung nach einem der Ansprüche 1 bis 9.

**11.** Parfümiertes Produkt nach Anspruch 10 in Form einer Seife, eines Dusch- oder Badegels, eines Shampoos oder eines anderen Haarpflegeprodukts, eines Kosmetikpräparats, eines Körperdeodorants oder Antitranspirants, eines Raumlufterfrischers, eines Waschmittels oder Textilweichspülers oder eines Pflegeprodukts zur Verwendung im Haushalt oder in der Industrie.

**12.** Verwendung einer Zusammensetzung nach einem der Ansprüche 1 bis 10, um eine antimikrobielle Aktivität zu verleihen oder die antimikrobielle Aktivität eines zur Körper- oder Haarpflege vorgesehenen Artikels oder eines funktionellen Produkts zu verstärken, **dadurch gekennzeichnet, daß** man diesem Artikel oder Produkt eine Duftzusammensetzung nach einem der Ansprüche 1 bis 10 zusetzt.

**Claims**

**1.** An antimicrobial perfuming composition, **characterised in that** the said composition comprises

- at least 40% w/w of a perfuming ingredient having an antimicrobial activity of at least 80% as measured by the "agar surface coating test" (ASCT), by the "vapour phase test" (VPT), or by the "direct-spray method" (DSM); and
- at least 0.1 % of an active ingredient selected from the group consisting of grapefruit-pip extract, fumitory extract, fumaric acid, or an ester of fumaric or lactic acid.

**2.** A composition according to claim 1, **characterised in that** said composition comprises an antimicrobial agent selected from Triclocarban and Triclosan.

**3.** A composition according to claim 1 or 2, **characterised in that** the fumaric acid ester is diethyl fumarate or digeranyl fumarate.

**4.** A composition according to any one of claims 1 to 3, **characterised in that** said composition comprises a surfactant of an anionic, cationic, non-ionic or amphoteric nature, or a phospholipid.

**5.** A composition according to claim 4, **characterised in that** the surfactant is a phospholipid or a quaternary surfactant.

**6.** A composition according to any one of claims 1 to 5, **characterised in that** it comprises an emollient of current use in perfumery or cosmetics.

**7.** A composition according to claim 6, **characterised in that** the emollient is an ester of lactic or fumaric acid.

**8.** A composition according to any one of claims 1 to 7, **characterised in that** it contains at least 60% by weight of perfuming ingredients each having an antimicrobial activity of at least 80% as measured by the "ASCT" method or the "VPT" method and at least 0.1% by weight of an active ingredient as specified in claim 1.

9. A composition according to claim 8, **characterised in that** each perfuming ingredient exhibits an antimicrobial activity of 100% as measured by the "ASCT" method or by the "VPT" method.

10. A perfumed article containing a perfuming composition according to any one of claims 1 to 9.

11. By way of a perfumed article according to claim 10, a soap, a bath or shower gel, a shampoo or other hair-care product, a cosmetic preparation, a body deodorant or antiperspirant, an air freshener, a detergent or textile softener, or a cleaning product for domestic or industrial use.

12. Use of a composition according to any one of claims 1 to 10 to confer an antimicrobial activity or to increase the antimicrobial activity of an article intended for body care or hair care, or of a functional product, **characterised in that** a perfuming composition according to any one of claims 1 to 10 is added to this article or product.